# FASCICULE DE BREVET EUROPEEN

(11) **EP 0 880 599 B1**
(45) Date de publication et mention de la délivrance du brevet: **11.06.2003**
(21) Numéro de dépôt: 97935649.0
(22) Date de dépôt: 29.07.1997
(51) Int. Cl.: C12Q 1/04, C12Q 1/37

(54) **PROCEDE DE MISE EN EVIDENCE D'UNE ACTIVITE ENZYMATIQUE DE MICROORGANISMES**
VERFAHREN ZUM NACHWEIS DER ENZYMAKTIVITÄT VON MIKROORGANISMEN
METHOD FOR DEMONSTRATING AN ENZYMATIC ACTIVITY OF MICRO-ORGANISMS

(30) Priorité: 29.07.1996 FR 9609523
(43) Date de publication de la demande: 02.12.1998
(73) Titulaire: BIO MERIEUX, 69280 Marcy l'Etoile (FR)
(72) Inventeur: ORENGA, Sylvain, F-01160 Neuville sur Ain (FR)
(74) Mandataire: Tonnellier, Jean-Claude
(86) Numéro de dépôt international: FR9701415
(87) Numéro de publication internationale: WO98004735

(56) Documents cités:
- WO-A-90/12888
- WO-A-92/12259
- Z. LOJDA ET AL.: "The histochemical demonstration of aminopeptidase with Bromoindolyl Leucinamide." HISTOCHEMISTRY, vol. 43, 1975, pages 355-366, XP000671121 cité dans la demande
- J. RATH ET AL.: "Ectoenzymatic activity and bacterial dynamics along a trophic gradient in the caribbean sea." MARINE ECOLOGY PROGRESS SERIES, vol. 102, 1993, pages 89-96, XP000671132

## Description

La présente invention concerne un procédé de mise en évidence d'une activité enzymatique de microorganismes. Un tel procédé peut être utilisé pour l'identification de microorganismes exprimant ou n'exprimant pas cette activité enzymatique.

La détection et l'identification des microorganismes sont très importantes notamment en médecine, dans l'industrie agro-alimentaire, pour le contrôle de l'environnement (par exemple le contrôle de l'eau...). Les microorganismes peuvent être recherchés pour leur pathogénicité, comme indicateurs de contamination, ou encore pour le contrôle de procédés de fabrication.

Les techniques de détection et d'identification de microorganismes sont actuellement basées sur la recherche de séquence nucléotidiques caractéristiques, la recherche d'antigènes ou d'anticorps, la culture, en milieu sélectif ou non sélectif, ou encore sur la recherche d'activités métaboliques et notamment enzymatiques (par exemple activités d'osidases, d'estérases, de peptidases, d'oxydases, etc.).

Le plus souvent, les procédés de détection et d'identification des microorganismes associent plusieurs de ces techniques. La culture est ainsi utilisée pour multiplier et sélectionner les microorganismes recherchés. Afin de simplifier leur détection, il a été proposé de mettre en évidence des activités biochimiques en introduisant des molécules produisant une coloration ou une fluorescence, directement dans le milieu de culture. De tels milieux sont appelés milieux de détection. Les activités biochimiques peuvent être mises en évidence par diverses méthodes telles que :
- la modification physico-chimique du milieu : changement de pH révélé à l'aide d'un indicateur coloré ou fluorescent (méthyl-umbelliférone).
- le changement du potentiel redox révélé à l'aide d'un indicateur coloré (sel de tétrazolium) ou fluorescent,
- la réaction d'une molécule produite par les microorganismes avec un composé présent dans le milieu, conduisant à une coloration,
- l'hydrolyse de molécules libérant un composé coloré ou fluorescent (naphtol, coumarine).

Les hydrolyses détectées sont en général le résultat de l'action d'une enzyme produite par le microorganisme sur un substrat enzymatique naturel ou synthétique. Ces activités enzymatiques sont par exemple celles des enzymes suivantes : estérases (par exemple lipases, phosphatases), osidases (β-galactosidase, β-glucuronidase, N-acétyl-hexosaminidase), peptidases (alanine-aminopeptidase, trypsinase, gélatinase), DNAses, décarboxylases, désaminases, uréases, tryptophanases, oxydases, catalases, etc.

On sait que les milieux gélifiés sont particulièrement bien adaptés à la culture et à l'isolement de microorganismes à partir d'un prélèvement, ainsi qu'à la détection de microorganismes "cibles" au sein d'un mélange de microorganismes. Sur ces milieux, les microorganismes forment des colonies détectables à l'oeil nu, et il est hautement souhaitable que les produits des activités biochimiques recherchées restent localisés sur leur site de production. Cela permet en effet de distinguer une colonie de ses voisines si elles n'expriment pas les mêmes activités. On peut ainsi utiliser diverses méthodes détectant par exemple des changements de pH (FR-A-2 671 100), des activités d'estérases (FR-2 457 323), des activités d'osidases (FR-A-2 684 110) etc. Il est évidemment possible d'utiliser conjointement plusieurs de ces méthodes, afin de mettre en évidence plusieurs espèces ou souches, et/ou d'accroître la sensibilité et/ou la spécificité de la détection.

On ne dispose pas actuellement de moyen, adapté aux milieux gélifiés, pour mettre en évidence des activités de L-Alanine-aminopeptidases, D-Alanine-aminopeptidases et L-Leucine-aminopepcidases de microorganismes. En effet, les substrats enzymatiques utilisés jusqu'à présent libèrent des molécules colorées ou fluorescentes qui diffusent dans les milieux gélifiés et/ou qui ne sont révélées que par irradiation U.V. (cas de la naphtylamine ou de l'aminocoumarine) et/ou après action de réactifs (cas de la naphtylamine), ou dont la coloration est peu contrastée dans les milieux réactionnels utilisés en microbiologie (cas de la nitroaniline).

On sait que la L-Leucine-aminopeptidase a été mise en évidence dans des coupes histologiques de mammifères grâce à un substrat enzymatique, la L-Leucine-3-(5-Bromoindolamine), par abréviation L-Leu-BIA, qui produit après hydrolyse un composé coloré ; voir Pearson *et al*., 1963, Lab. Invest., **12** : 712. En 1967, Yarborough *et al*., J. Reticuloendoth. Soc., **4** : 390 ont repris la technique de Pearson *et al*. dans des applications similaires (coupes histologiques). Ils ont précisé que l'ajout d'un mélange de ferri- et ferro-cyanure de potassium ou de sulfate de cuivre inhibe la réaction.

En 1975 Lojda et Havránková, Histochemistry, **43** : 355 proposèrent d'améliorer la méthode utilisant le substrat L-Leu-BIA par l'addition d'un mélange de sel de tétrazolium et de phénazine méthosulfate, la réaction colorée observée provenant alors de la réduction du sel de tétrazolium en formazan.

Lors des travaux ayant conduit à la présente invention, on a recherché s'il était possible d'utiliser la L-Leu-BIA comme substrat enzymatique dans la détection de microorganismes cultivés notamment sur milieux gélifiés. Lors d'essais préliminaires, on a ajouté de la L-Leu-BIA dans le milieu qui est décrit à l'exemple 1 ci-après. Ce milieu est couramment utilisé pour la recherche d'osidases.

Il n'a pas été possible de mettre en évidence une activité de peptidase, quel que soit le microorganisme cultivé dans ce milieu (*Escherichia coli, Klebsiella, Citrobacter, Pseudomonas, Enterococcus, Staphylococcus, Streptococcus*).

En revanche, si l'on ajoute dans ce même milieu de la L-Leucine-7-amino-4-méthyl-coumarine (L-Leu-AMC), une fluorescence est détectée avec certains de ces mêmes microorganismes. De même dans ce milieu, avec des substrats d'osidases (5-Bromo-4-chloro-3-indolyl-β-D-galactoside et 6-Chloro-3-indolyl-β-D-glucuronide), on peut détecter les activités de β-galactosidase et de β-glucuronidase des microorganismes. L'ajout des réactifs proposés par Lojda et Havránková s'est traduit par une inhibition plus ou moins complète de la croissance des microorganismes sans permettre la révélation d'une activité de peptidase avec la L-Leu-BIA.

De même, avec le milieu utilisé à l'exemple 2 ci-après, on n'a pas pu mettre en évidence une activité de peptidase avec L-Leu-BIA, alors que le substrat L-Leu-AMC permet de détecter cette activité dans le même milieu.

On a maintenant découvert que l'absence de résultats avec les dérivés de BIA n'était pas due à une incompatibilité avec les microorganismes ou à une inhibition de leur multiplication en culture, mais était due essentiellement à des conditions de milieu. On a en effet découvert qu'il était possible de révéler l'activité de peptidase de microorganismes avec la L-Leu-BIA, en utilisant d'autres milieux de culture. Les raisons pour lesquelles certains milieux sont utilisables, et d'autres non, ne sont pas connues. Néanmoins, il est possible de déterminer et de mettre au point, par de simples expériences de routine, semblables à celles décrites dans la partie expérimentale ci-après, les milieux et/ou ingrédients qui conviennent ou qui ne conviennent pas. L'invention a donc d'abord consisté notamment à rechercher, et à montrer qu'il était possible de trouver, des milieux de culture dans lesquels les substrats de peptidases dérivés de la 5-bromoindolamine mentionnés ci-dessus sont utilisables pour mettre en évidence les activités enzymatiques correspondantes dans une culture de microorganismes.

On a ainsi découvert notamment que le milieu suivant est utilisable :
- Extrait de levure 0,5-25 g/l
- Peptone de gélatine 0,5-25 g/l
- NaCl 0-50 g/l
et éventuellement :
- Agent régulateur de pH, q.s.p. pH = 3 à 9
et/ou :
- Agent gélifiant 5-35 g/l

Le pH choisi est un pH qui convient pour le microorganisme étudié. Dans le cas d'un milieu gélifié, le pH est de préférence de 5 à 9. On peut ajuster le pH, par exemple, avec de l'acide chlorhydrique ou du carbonate de sodium.

Si on ajoute à un tel milieu de la L-Leu-BIA, et si on ensemence avec des microorganismes, on obtient après culture des colonies brunes ou incolores suivant que les microorganismes expriment ou non une activité de L-Leucine-aminopeptidase.

Des résultats comparables ont été obtenus avec les substrats L-Ala-BIA et D-Ala-BIA.

L'invention a donc pour objet l'utilisation d'au moins un composé de formule :

X-NH-R

dans laquelle X représente un groupe 5-bromoindole-3-yle et R représente le reste acyle d'un acide aminé choisi parmi la leucine et l'alanine,
comme agent révélateur permettant de mettre en évidence, par formation d'un produit coloré, une activité de peptidase dans une culture de microorganismes.

En particulier le groupe R représente un reste acyle de L-Leu, L-Ala ou D-Ala.

Les substrats dont l'utilisation fait l'objet de la présente demande n'inhibent pas la multiplication des microorganismes dans les milieux de culture appropriés. On peut donc utiliser l'agent révélateur en l'ajoutant au milieu de culture des microorganismes, avant le début de la culture ou en début de culture.

L'un des avantages importants des agents révélateurs utilisés selon l'invention est qu'en présence de l'activité de peptidase recherchée, ils donnent des produits colorés qui ne diffusent pas dans le milieu gélifié.

Ils peuvent donc être utilisés en milieu gélifié. Ils peuvent aussi, bien entendu, être utilisés en milieu liquide.

Selon un mode de réalisation particulier, on peut ajouter à la culture, en outre, au moins un autre agent révélateur permettant de mettre en évidence, par formation d'un produit coloré ou fluorescent, une activité enzymatique généralement différente de celle qui est mise en évidence à l'aide des composés de formule X-NH-R tels que définis ci-dessus. Il peut s'agir par exemple d'une activité d'estérase, d'osidase ou de peptidase. On peut ainsi obtenir des informations supplémentaires, en liaison avec une absence de coloration (ou de fluorescence) ou en liaison avec une coloration modifiée par rapport à la coloration obtenue avec un seul substrat enzymatique. L'autre agent révélateur choisi aura des propriétés différentes de celles des dérivés de BIA : par exemple, on choisira un autre agent révélateur capable de donner un produit réactionnel ayant une couleur différente de la couleur brune obtenue avec les dérivés de BIA. L'autre agent révélateur (ou second agent révélateur) permettra donc de révéler, grâce à sa couleur propre, ou grâce à sa fluorescence, la présence d'une activité enzymatique dont il est spécifique. Si l'activité de peptidase révélable par les dérivés de BIA est également présente, on obtiendra une coloration modifiée, différente de ladite couleur brune et différente aussi de ladite couleur propre donnée par le second agent révélateur. Des exemples d'utilisation de plusieurs substrats, ainsi que les informations qui peuvent en ressortir, sont donnés ci-après dans la partie expérimentale. Bien entendu, les résultats peuvent varier avec chaque espèce ou souche de microorganisme étudié.

Chaque cas susceptible d'intérêt doit donc faire l'objet d'études préalables selon des expériences de routine.

Les dérivés servant à mettre en évidence des activités enzymatiques différentes, et utilisables comme autres agents révélateurs, sont notamment des dérivés d'indoxyle, de coumarine, de résorufine, de naphtol, de naphtylamine, de nitrophénol, de nitroaniline, de rhodamine, d'hydroxyquinoléine, de fluorescéine, etc.

Parmi ces dérivés qui peuvent être utilisés en association avec les dérivés de BIA, on peut citer notamment le 5-bromo-4-chloro-3-indolyl-β-D-glucuronide, le 6-chloro-3-indolyl-β-D-glucoside, la L-alanine-7-amino-4-méthyl-coumarine, le 4-méthyl-umbelliféryl-N-acétyl-β-D-galactosaminide, le résorufine-β-D-galac-toside, le sulfate de β-naphtyle, le naphtol AS-BI β-D-galactoside, le L-alanine β-naphtylamide, l'o-nitrophénol-β-D-galactoside, le carboxybenzoyle-L-arginine-p-nitroanilide, la rhodamine 110 bis-(L-leucine amide), l'hydroxyquinoléine-β-D-glucoside, et le diacétate de fluorescéine.

L'invention a également pour objet un procédé de mise en évidence d'une activité de peptidase dans une culture de microorganisme, dans lequel on ajoute au milieu de culture desdits microorganismes un agent révélateur permettant de mettre en évidence ladite activité par formation d'un produit coloré, caractérisé par le fait que ledit agent révélateur comprend au moins un composé de formule :

X-NH-R,

dans laquelle X et R sont définis comme ci-dessus.

On peut ajouter le composé X-NH-R avant le début de la culture, ou en cours de culture, ou même en fin de culture. La mise en oeuvre du procédé de l'invention implique donc de cultiver les microorganismes étudiés, étant entendu que l'on peut effectuer cette culture avant ou après l'addition du composé X-NH-R, et que l'on peut également effectuer cette addition en cours de culture. Bien entendu, on effectue la culture proprement dite par incubation d'un milieu de culture approprié dans des conditions permettant la multiplication des microorganismes étudiés. Les compositions des milieux de culture et les conditions de culture qui conviennent sont connues ou peuvent être déterminées par des expériences de routine.

L'invention a également pour objet un milieu de culture de microorganismes contenant, outre les ingrédients nécessaires à la culture desdits microorganismes, au moins un composé de formule X-NH-R.

Les dérivés de formule X-NH-R sont utilisés à des concentrations suffisantes pour donner des réactions colorées observables. Ces concentrations, qui peuvent être déterminées par expériences de routine, peuvent varier généralement de 25 à 2000 mg par litre de milieu de culture.

Les caractéristiques et avantages de l'invention sont illustrés par les exemples suivants.

### EXEMPLES

### Exemple 1

Le milieu de culture contient, outre de l'eau :
- Peptone de viande * 15 g/l
- Peptone de caséine ** 3 g/l
- NaCl 5 g/l
- Tampon Tris 0,5 g/l
- Na₂HPO₄ 1 g/l
- Citrate de sodium 1 g/l
- Glucose 1 g/l
- Pyruvate de sodium 2 g/l
- Agar 13 g/l

* commercialisé par : D.I.F.C.O.
** commercialisé par : D.I.F.C.O.

A ce milieu, on ajoute soit de la L-Ala-BIA, soit de la L-Leu-BIA, soit de la L-Ala-AMC soit de la L-Leu-AMC, à des concentrations de 200 mg/l. On ensemence les différents milieux obtenus, répartis dans des boîtes de Petri, avec des microorganismes. Les boîtes ont été mises en incubation à 37°C pendant 48 heures. Les colonies formées ont été examinées visuellement à la lumière ambiante et sous lampe U.V. (longueur d'onde = 365 nm) après 24 et 48 heures d'incubation. La couleur ou la présence de fluorescence ont été notées. Les microorganismes étudiés étaient : *Escherichia coli, Klebsiella pneumoniae, Citrobacter freundii, Pseudomonas aeruginosa, Streptococcus agalactiae, Enterococcus faecium, Streptococcus pyogenes, Staphylococcus epidermidis et Candida albicans*. Les résultats sont présentés dans le tableau I ci-après :

**TABLEAU I**

| Souches | | L-Ala-AMC | L-Ala-SIA | L-Leu-AMC | L-Leu-BIA |
|---|---|---|---|---|---|
| *E. coli* | 24 H | Fluo¹ | ―² | Fluo | ― |
| | 48 H | Fluo | ― | Fluo | ― |
| *K. pneumoniae* | 24 H | Fluo | ― | Fluo | ― |
| | 48 H | Fluo | ― | Fluo | ― |
| *C. freundii* | 24 H | Fluo | ― | Fluo | ― |
| | 48 H | Fluo | ― | Fluo | ― |
| *P. aeruginosa* | 24 H | Fluo | ― | ― | ― |
| | 48 H | Fluo | ― | Fluo | ― |
| *S. agalactiae* | 24 H | ― | ― | Fluo | ― |
| | 48 H | Fluo | ― | Fluo | ― |
| *E. faecium* | 24 H | ― | ― | ― | ― |
| | 48 H | Fluo | ― | Fluo | ― |
| *S. pyogenes* | 24 H | ― | ― | Fluo | ― |
| | 48 H | Fluo | ― | Fluo | ― |
| *S. epidermidis* | 24 H | *―* | *―* | *―* | ― |
| | 48 H | ― | ― | ― | ― |
| *C. albicans* | 24 H | ― | ― | NT³ | NT |
| | 48 H | Fluo | ― | NT | NT |

| | | | | | |
|---|---|---|---|---|---|
| ¹ : **Fluo** = Fluorescence | | | | | |
| ² : **―** = absence de fluorescence et/ou absence de coloration | | | | | |
| ³ : **NT** = non testé | | | | | |

Le milieu utilisé dans cet exemple permet de mettre en évidence les activités L-Alanine-aminopeptidase et L-Leucine-aminopeptidase avec les réactifs L-Ala-AMC et L-Leu-AMC, respectivement, mais lorsqu'on utilise la L-Ala-BIA, et la L-Leu-BIA aucune hydrolyse n'est détectée.

### Exemple 2

Dans un tampon phosphate 0,1 M pH 7,3 à 25°C on ajoute soit de la L-Leu-BIA soit de la L-Leu-AMC, à des concentrations de 400 mg/l. Les milieux obtenus ont été répartis dans les puits de plaques de microtitration et ensemencés avec des suspensions de microorganismes. Les plaques ont été mises en incubation 24 heures à 37°C. Les puits ont été examinés visuellement à la lumière ambiante et sous lampe U.V., comme précédemment. Après 24 et 48 heures d'incubation, la couleur ou la présence de fluorescence ont été notées. Les résultats sont présentés dans le tableau II ci-après :

**TABLEAU II**

| Souches | | L-Leu-AMC | L-Leu-BIA |
|---|---|---|---|
| *E. coli* | 24 H | Fluo¹ | ―² |
| | 48 H | Fluo | ― |
| *K. pneumoniae* | 24 H | Fluo | ― |
| | 48 H | Fluo | ― |
| *C. freundii* | 24 H | Fluo | ― |
| | 48 H | Fluo | ― |
| *P. aeruginosa* | 24 H | ― | ― |
| | 48 H | Fluo | ― |
| *S. agalactiae* | 24 H | Fluo | ― |
| | 48 H | Fluo | ― |
| *E. faecium* | 24 H | ― | ― |
| | 48 H | Fluo | ― |
| *S. pyogenes* | 24 H | Fluo | ― |
| | 48 H | Fluo | ― |
| *S. epidermidis* | 24 H | ― | ― |
| | 48 H | ― | ― |

| | | | |
|---|---|---|---|
| ¹ : **Fluo** = Fluorescence | | | |
| ² : **―** = absence de fluorescence et/ou absence de coloration | | | |

Ainsi, avec le milieu utilisé dans cet exemple, il est possible de mettre en évidence l'activité de L-Leucine-aminopeptidase avec L-Leu-AMC, mais lorsqu'on utilise la L-Leu-BIA aucune hydrolyse n'est détectée.

### Exemple 3

Le milieu de culture contient, outre de l'eau :
- Extrait de levure * 6 g/l
- Peptone de gélatine ** 5 g/l
- NaCl 8 g/l
- Na₂CO₃ 0,1 g/l
- Agar 13 g/l

* commercialisé par BIO MERIEUX
** BIO-GELYTONE commercialisé par BIO MERIEUX

A ce milieu on ajoute soit de la L-Ala-BIA, soit de la L-Leu-BIA, soit de la L-Ala-AMC, soit de la L-Leu-AMC, à des concentrations de 200 mg/l. On ensemence les différents milieux obtenus, répartis en boîtes de Petri, avec les mêmes microorganismes que ceux utilisés dans l'exemple 1. Les boîtes sont mises en incubation à 37°C pendant 48 heures. Les colonies formées ont été examinées visuellement à la lumière ambiante et sous lampe U.V. (longueur d'onde = 365 nm) après 24 et 48 heures d'incubation. La couleur ou la présence de fluorescence ont été notées. Les résultats sont présentés dans le tableau III ci-après :

**TABLEAU III**

| Souches | | L-Ala-AMC | L-Ala-BIA | L-Leu-AMC | L-Leu-BIA |
|---|---|---|---|---|---|
| *E. coli* | 24 H | Fluo¹ | Brun | Fluo | ―² |
| *Gram négatif* | 48 H | Fluo | Brun | Fluo | Brun |
| *K. pneumoniae* | 24 H | Fluo | Brun | Fluo | ― |
| *Gram négatif* | 48 H | Fluo | Brun | Fluo | Brun |
| *C. freundii* | 24 H | Fluo | Brun | Fluo | Brun |
| *Grain négatif* | 48 H | Fluo | Brun | Fluo | Brun |
| *P. aeruginosa* | 24 H | Fluo | Brun | ― | ― |
| *Gram négatif* | 48 H | Fluo | Brun | Fluo | Brun |
| *S. agalactiae* | 24 H | ― | ― | Fluo | ― |
| *Gram positif* | 48 H | Fluo | Brun | Fluo | ― |
| *E. faecium* | 24 H | ― | ― ― ― | ― | ― |
| *Gram positif* | 48 H | Fluo | Brun | Fluo | ― |
| *S. pyogenes* | 24 H | ― | ― | Fluo | ― |
| *Gram positif* | 48 H | Fluo | Brun | Fluo | ― |
| *S. epidermidis* | 24 H | ― | ― | ― | ― |
| *Gram positif* | 48 H | ― | ― | ― | ― |
| *C. albicans* | 24 H | ― | ― | NT³ | NT |
| | 48 H | Fluo | Brun | NT | NT |

| | | | | | |
|---|---|---|---|---|---|
| ¹ : **Fluo** = Fluorescence | | | | | |
| ² : **―** = absence de fluorescence et/ou absence de coloration | | | | | |
| ³ : **NT** = non testé | | | | | |

Le milieu utilisé dans cet exemple permet de mettre en évidence les activités de L-Alanine-aminopeptidase et de L-Leucine-aminopeptidase avec la L-Ala-AMC et la L-Leu-AMC, respectivement, ainsi qu'avec la L-Ala-BIA et la L-Leu-BIA. Ces deux derniers substrats donnent cependant des réactions parfois plus tardives. En revanche, ils permettent de distinguer les bactéries à Gram négatif des bactéries à Gram positif. En effet :
- après 24 heures d'incubation avec L-Ala-BIA, les bactéries Gram positif ne donnent pas de coloration, alors que les bactéries Gram négatif donnent une coloration brune ;
- et après 48 heures avec la L-Leu-BIA : les bactéries Gram positif ne donnent pas de coloration, alors que les bactéries Gram négatif donnent une coloration brune.

### Exemple 4

Dans le milieu de l'exemple 3, on a ajouté soit de la L-Leu-BIA soit de la L-Ala-BIA, seules ou en combinaison avec soit du 5-bromo-4-chloro-3-indolyl-β-D-glucoside (X-Glu), soit du 6-chloro-3-indolyl-β-D-glucoside (Z-Glu), soit du 4-méthylumbel-liféryl-β-D-glucoside (MUGI) soit du 5-Bromo-4-chloro-3-indolyl-acétate (X-Ac).

Les concentrations de ces différents substrats sont les suivantes :
- L-Leu-BIA : 200 mg/l
- L-Ala-BIA : 200 mg/l
- X-Glu : 200 mg/l
- Z-Glu : 200 mg/l
- MuGl : 200 mg/l

On ensemence ces différents milieux répartis dans des boîtes de Petri avec des microorganismes. Les souches étudiées sont les mêmes qu'aux exemples précédents. Les boîtes ont été mises en incubation à 37°C pendant 48 heures, et les colonies formées ont été examinées visuellement à la lumière ambiante et sous lampe U.V. (longueur d'onde = 365 nm). Les résultats d'observation des couleurs des colonies obtenues après 24 et 48 heures d'incubation sont présentés dans le tableau IV :

**TABLEAU IV**

| | | L-Ala-BIA | | | | | L-Leu-BIA | | | | |
|---|---|---|---|---|---|---|---|---|---|---|---|
| | | ― | X-Glu | Z-Glu | MUGI | X-Ac | ― | X-Glu | Z-Glu | MUGI | X-Ac |
| Souches | N° du milieu | 1 | 2 | 3 | 4 | 5 | 6 | 7 | 8 | 9 | 10 |
| *E. coli* | 24 H | Brun | Brun | Brun | Brun | Brun | ― | ― | ― | ― | ― |
| *Gram négatif* | 48 H | Brun | Brun | Brun | Brun | Brun | Brun | Brun | Brun | Brun | Gris- |
| | | | | | | | | | | | Brun |
| *K. pneumoniae* | 24 H | Brun | Gns | Gris-Rose | Brun+ Fluo | Gris | ― | Bleu | Gris-Rose | Fluo | Gris-Brun |
| *Gram négatif* | 48 H | Brun | Gris | Gris-Rose | Brun+ Fluo | Gris-Brun | Brun | Bleu-Gris | Gris-Rose | Brun+Fluo | Gris-Brun |
| *C. freundii* | 24 H | Brun | Gris | Gris-Rose | Brun+ Fluo | Gris | Brun | Gris | Gris-Rose | Brun+ Fluo | Gris-Brun |
| *Gram négatif* | 48 H | Brun | Gris | Gris-Rose | Brun+ Fluo | Brun | Brun | Gris | Gris-Rose | Brun+ Fluo | Gris-Brun |
| *P. aeruginosa* | 24 H | Brun | Brun | Brun | Brun | Gris-Brun | ― | ― | ― | ― | Gris |
| *Gram négatif* | 48 H | Brun | Brun | Brun | Brun | Gris-Brun | Brun | Brun | Brun | Brun | Gris-Brun |
| *S. agalactiae* | 24 H | ― | ― | ― | ― | ― | ― | ― | ― | ― | ― |
| | 48 H | Brun | Brun | Brun | Brun | Gris | ― | ― | ― | ― | ― |
| *E. faecium* | 24 H | ― | Bleu | Rose | Fluo | Bleu | ― | Bleu | Rose | Fluo | Bleu |
| | 48 H | Brun | Gris-Bleu | Rose-Gris | Brun+ Fluo | Gris-Bleu | ― | Bleu | Rose | Fluo | Gris-Bleu |
| *S. pyogenes* | 24 H | ― | ― | ― | ― | ― | ― | ― | ― | ― | ― |
| | 48 H | Brun | Brun | Brun | Brun | Gris-Bleu | ― | ― | ― | ― | ― |
| *S. epidermidis* | 24 H | ― | ― | ― | ― | ― | ― | ― | ― | ― | ― |
| | 48 H | ― | ― | ― | ― | Turquoise | ― | ― | ― | ― | ― |

Sur les milieux 2, 3 et 4 il est possible de distinguer après 24 heures d'incubation les *Enterococcus* (*E. faecium*) qui seuls donnent une coloration autre que brune ou grise. Les milieux 2, 3 et 4 permettent aussi, après 24 heures de culture, de distinguer les bactéries Gram positif, qui présentent soit une absence de coloration soit une coloration autre que brune ou grise. Les milieux 7, 8 et 9 permettent des distinctions similaires, mais après 48 heures d'incubation. Sur le milieu 5 après 48 heures d'incubation seul *S. epidermidis* donne des colonies de couleur turquoise, les autres bactéries donnant des colonies brunes à gris-bleu.

On précise que lorsque les substrats ci-après sont présents seuls dans le milieu réactionnel, la présence d'une osidase et l'hydrolyse résultante du 5-bromo-4-chlororo-3-indolyl-β-D-gluco-side (X-Glu) entraîne l'apparition d'une couleur bleu turquoise, l'hydrolyse du 6-chloro-3-indolyl-β-D-glucoside (Z-Glu) entraîne l'apparition d'une couleur rose-pourpre. L'hydrolyse du 4-méthyl-umbelliféryl-β-D-glucoside (MUGI) entraîne l'apparition d'une fluorescence bleue sous lampe U.V. (longueur d'onde = 365 nm) ; et que, en présence d'une estérase, l'hydrolyse résultante du 5-bromo-4-chloro-3-indolyl-acétate (X-Ac) entraîne l'apparition d'une couleur bleu turquoise.

### Exemple 5

Le milieu de culture contient, outre de l'eau :
- Extrait de levure * 6 g/l
- Peptone de gélatine ** 5 g/l
- NaCl 8 g/l
- Na₂CO₃ 0,1 g/l

* commercialisé par BIO MERIEUX
** BIO-GELYTONE commercialisé par BIO MERIEUX

A ce milieu, on ajoute soit de la L-Leu-BIA soit de la L-Ala-BIA, seules, ou en combinaison entre elles, ou en combinaison avec du 5-Bromo-4-chloro-3-indolyl-acétate (X-Ac).

Les concentrations de ces différents substrats sont les suivantes :
- L-Leu-BIA : 300 mg/l
- L-Ala-BIA : 300 mg/l
- X-Ac : 200 mg/l

Les milieux obtenus ont été répartis dans des plaques de microtitration et ensemencés avec des suspensions de microorganismes, comme précédemment. Les plaques ont été mises en incubation 48 heures à 37°C. Les couleurs des puits obtenues après 24 et 48 heures d'incubation sont présentées dans le tableau V :

**TABLEAU V**

| | | L-Leu-BIA | L-Ala-BIA | L-Ala-BIA + L-Leu-BIA | L-Ala-BIA + X-Ac |
|---|---|---|---|---|---|
| Souches | N° du milieu | 1 | 2 | 3 | 4 |
| *E. coli* | 24 H | ― | Brun | Brun | Gris |
| | 48 H | Brun | Brun | Brun | Gris |
| *K. pneumoniae* | 24 H | Brun | Brun | Brun | Gris |
| | 48 H | Brun | Brun | Brun | Gris |
| *C. freundii* | 24 H | Brun | Brun | Brun | Gris |
| | 48 H | Brun | Brun | Brun | Gris |
| *P. aeruginosa* | 24 H | Brun | Brun | Brun | Gris |
| | 48 H | Brun | Brun | Brun | Gris |
| *S. agalactiae* | 24 H | ― | ― | Brun | Gris-Bleu |
| | 48 H | ― | Brun | Brun | Gris-Bleu |
| *E. faecium* | 24 H | ― | ― | Brun | Gris-Bleu |
| | 48 H | ― | ― | Brun | Gris-Bleu |
| *S. pyogenes* | 24 H | ― | ― | Brun | Gris-Bleu |
| | 48 H | Brun | Brun | Brun | Gris-Brun |
| *S. epidermidis* | 24 H | ― | ― | ― | Turquoise |
| | 48 H | ― | ― | ― | Turquoise |

On voit qu'avec les milieux 3 et 4 il est possible de distinguer *S. epidermidis* des autres bactéries. Avec le milieu 1 il est possible de différencier des bactéries à Gram négatif de celles à Gram positif (sauf *S. pyogenes*) après 48 heures d'incubation. Le milieu 2 permet la différenciation des bactéries Gram positif et Gram négatif après 24 heures d'incubation.

### Exemple 6

Le milieu de culture contient, outre de l'eau :
- Extrait de viande de boeuf * 3 g/l
- Peptone de gélatine ** 5 g/l
- NaCl 8 g/l
- Agar 15 g/l

* commercialisé par BIO MERIEUX
** BIO-GELYTONE (BIO MERIEUX)

On ajoute à ce milieu soit de la L-Ala-BIA, soit de la L-Ala-AMC, à des concentrations de 300 mg/l et de 200 mg/l, respectivement. On ensemence les différents milieux obtenus répartis en boîtes de Petri, avec des microorganismes. Les boîtes sont mises à incubation à 37°C pendant 48 heures. Les colonies formées ont été examinées visuellement à la lumière ambiante et sous lampe U.V. comme précédemment, après 24 et 48 heures d'incubation. La couleur ou la présence de fluorescence ont été notées. Les résultats sont présentés dans le tableau VI ci-après.

**TABLEAU VI**

| Souches | | L-Ala-AMC | L-Ala-BIA |
|---|---|---|---|
| *E. coli* | 24 H | Fluo¹ | Brun |
| | 48 H | Fluo | Brun |
| *K. pneumoniae* | 24 H | Fluo | Brun |
| | 48 H | Fluo | Brun |
| *C. freundii* | 24 H | Fluo | Brun |
| | 48 H | Fluo | Brun |
| *P. aeruginosa* | 24 H | Fluo | ― |
| | 48 H | Fluo | Brun |
| *S. agalactiae* | 24 H | ―² | ― |
| | 48 H | Fluo | ― |
| *E. faecium* | 24 H | ― | ― |
| | 48 H | Fluo | ― |
| *S. pyogenes* | 24 H | ― | ― |
| | 48 H | Fluo | Brun |
| *S. epidermidis* | 24 H | ― | ― |
| | 48 H | ― | ― |
| *C. albicans* | 24 H | ― | ― |
| | 48 H | Fluo | Brun |

| | | | |
|---|---|---|---|
| ¹ : **Fluo** = Fluorescence | | | |
| ² : **―** = absence de fluorescence et/ou absence de coloration | | | |

Avec le milieu utilisé dans cet exemple, il est donc possible de mettre en évidence l'activité de L-alanine-aminopeptidase avec L-Ala-AMC ainsi qu'avec L-Ala-BIA.

## Revendications

1. Utilisation d'au moins un composé de formule :
X-NH-R
dans laquelle X représente un groupe 5-bromoindole-3-yle et R représente le reste acyle d'un acide aminé choisi parmi la leucine et l'alanine,
comme agent révélateur permettant de mettre en évidence, par formation d'un produit coloré, une activité de peptidase dans une culture de microorganismes.

2. Utilisation selon la revendication 1, dans laquelle R représente un reste acyle de L-Leu, L-Ala ou D-Ala.

3. Utilisation selon l'une quelconque des revendications précédentes, dans laquelle ladite culture est réalisée en milieu gélifié.

4. Utilisation selon l'une quelconque des revendications 1 et 2, dans laquelle ladite culture est réalisée en milieu liquide.

5. Utilisation selon l'une quelconque des revendications précédentes, dans laquelle en outre on ajoute à ladite culture au moins un autre agent révélateur permettant de mettre en évidence, par formation d'un produit coloré ou fluorescent, une activité enzymatique différente de celle mise en évidence conformément à la revendication 1.

6. Procédé de mise en évidence d'une activité de peptidase dans une culture de microorganismes, dans lequel on ajoute au milieu de culture desdits microorganismes un agent révélateur permettant de mettre en évidence ladite activité par formation d'un produit coloré, **caractérisé par le fait que** ledit agent révélateur comprend au moins un composé de formule :
X-NH-R
dans laquelle X représente un groupe 5-bromoindole-3-yle et R représente le reste acyle d'un acide aminé choisi parmi la leucine et l'alanine.

7. Milieu de culture de microorganismes contenant, outre les ingrédients nécessaires à la culture desdits microorganismes, au moins un composé de formule :
X-NH-R
dans laquelle X représente un groupe 5-bromoindole-3-yle et R représente le reste acyle d'un acide aminé choisi parmi la leucine et l'alanine.

8. Milieu de culture selon la revendication 7, contenant de 25 à 2000 mg/l dudit composé.

9. Milieu de culture selon la revendication 7 ou 8, **caractérisé par le fait qu'**il s'agit d'un milieu gélifié.

## Patentansprüche

1. Verwendung mindestens einer Verbindung der Formel:
X-NH-R
worin X für eine 5-Bromindol-3-yl-Gruppe steht und R den Acylrest einer Aminosäure darstellt, ausgewählt unter Leucin und Alanin,
als Indikator, der den Nachweis einer Peptidase-Aktivität in einer Mikroorganismenkultur durch Bildung eines gefärbten Produkts gestattet.

2. Verwendung gemäß Anspruch 1, worin R einen Acylrest von L-Leu, L-Ala oder D-Ala darstellt.

3. Verwendung nach einem der vorstehenden Ansprüche, worin die Kultur in einem gelierten Medium erfolgt.

4. Verwendung nach einem der Ansprüche 1 und 2, worin die Kultur in flüssigem Medium erfolt.

5. Verwendung nach einem der vorstehenden Ansprüche, worin man zu der Kultur zusätzlich mindestens einen weiteren Indikator zusetzt, der den Nachweis einer anderen enzymatischen Aktivität als derjenigen, die gemäß Anspruch 1 nachgewiesen wird, durch Bildung eines gefärbten oder fluoreszierenden Produktes gestattet.

6. Verfahren zum Nachweis einer Peptidase-Aktivität in einer Mikroorganismenkultur, bei dem man zum Kulturmedium der Mikroorganismen einen Indikator zugibt, welcher den Nachweis der Aktivität durch Bildung eines gefärbten Produktes gestattet, **dadurch gekennzeichnet, dass** der Indikator mindestens eine Verbindung der Formel
X-NH-R
umfasst, worin X für eine 5-Bromindol-3-yl-Gruppe steht und R einen Acylrest einer Aminosäure darstellt, ausgewählt unter Leucin und Alanin.

7. Kulturmedium für Mikroorganismen, umfassend zusätzlich zu den notwendigen Bestandteilen der Mikroorganismenkultur mindestens eine Verbindung der Formel:
X-NH-R
worin X eine 5-Bromindol-3-yl-Gruppe darstellt und R den Acylrest einer Aminosäure darstellt, ausgewählt unter Leucin und Alanin.

8. Kulturmedium gemäß Anspruch 7, enthaltend 25 bis 2000 mg/l der Verbindung.

9. Kulturmedium gemäß Anspruch 7 oder 8, **dadurch gekennzeichnet, dass** es sich um ein geliertes Medium handelt.

## Claims

1. Use of at least one compound of formula:
X-NH-R
in which X represents a 5-bromoindol-3-yl group and R represents the acyl residue of an amino acid chosen from leucine and alanine,
as a tracer which makes it possible to demonstrate, by formation of a coloured product, a peptidase activity in a microorganism culture.

2. Use according to Claim 1, in which R represents an acyl residue of L-Leu, L-Ala or D-Ala.

3. Use according to either of the preceding claims, in which the said culture is prepared in a gelled medium.

4. Use according to either of Claims 1 and 2, in which the said culture is prepared in a liquid medium.

5. Use according to any one of the preceding claims, in which, in addition, at least one other tracer which makes it possible to demonstrate, by formation of a coloured or fluorescent product, an enzymatic activity different from the one demonstrated in accordance with Claim 1, is added to the said culture.

6. Method for demonstrating a peptidase activity in a microorganism culture, in which a tracer which makes it possible to demonstrate the said activity, by formation of a coloured product, is added to the culture medium of the said microorganisms, **characterized in that** the said tracer comprises at least one compound of formula:
X-NH-R
in which X represents a 5-bromoindol-3-yl group and R represents the acyl residue of an amino acid chosen from leucine and alanine.

7. Microorganism culture medium containing, besides the ingredients necessary for culturing the said microorganisms, at least one compound of formula:
X-NH-R
in which X represents a 5-bromoindol-3-yl group and R represents the acyl residue of an amino acid chosen from leucine and alanine.

8. Culture medium according to Claim 7, containing from 25 to 2000 mg/l of the said compound.

9. Culture medium according to Claim 7 or 8, **characterized in that** it is a gelled medium.
